# EUROPEAN PATENT SPECIFICATION

(11) **EP 0 501 714 B1**
(45) Date of publication and mention of the grant of the patent: **02.05.1997**
(21) Application number: 92301511.9
(22) Date of filing: 24.02.1992
(51) Int. Cl.: A61K 7/48, A61K 7/42

(54) **Cationic compositions for skin**
Kationische Hautmittel
Compositions cationiques pour la peau

(30) Priority: 28.02.1991 US 662880; 28.02.1991 US 662680
(43) Date of publication of application: 02.09.1992
(73) Proprietor: UNILEVER PLC, London EC4P 4BQ (GB); UNILEVER N.V., 3013 AL Rotterdam (NL)
(72) Inventor: Ziegler, Philip Dale, Oxford, CT 06483 (US); Cheney, Michael Charles, Fairfield, CT 06430 (US)
(74) Representative: Evans, Jacqueline Gail Victoria

(56) References cited:
- EP-A- 0 013 713
- EP-A- 0 117 135
- EP-A- 0 381 618
- GB-A- 2 055 119
- SOAP,COSMETICS,CHEMICAL SPECIALTIES vol. 63, no. 7, July 1987, NEW YORK US page 87 'MOISTURIZING SUN BLOCK'

## Description

This invention relates to compositions which are suitable for application to human skin, including emollient compositions and waterproof sunscreen compositions.

Hosts of commercial emollient creams and lotions claim alleviation of the signs and symptoms of dry skin. Application of these products is intended to return skin to a normal condition by moisturizing and reducing evaporation of water from the skin. Most dry skin products are oil-in-water anionic or non-ionic emulsions. As a result, these products wash off with water and then must be re-applied.

Cationic emulsions occupy a much smaller market segment, but can possess certain skin feel advantages. In U.S. Patent 4,389,418 (Burton) there is disclosed a water-out emulsion containing petrolatum or a mineral oil to moisturize the skin, a quaternary ammonium emulsifier, a fatty alcohol and a fatty ester emollient. Greaseless skin conditioning compositions based upon cationic polymers have also been reported in U.S. Patent 4,438,095 (Grollier). Among the polymers disclosed are polyamines, polyaminoamides or quaternary polyammonium compounds. Emulsions are formed wherein the aqueous phase contains the cationic polymer and there are no detergent or foaming agents present.

Diquaternary nitrogen compounds have been reported in U.S. Patent 4,886,890 (Chaudhuri et al) as useful in skin lotions and shampoos. These compositions may be formulated with a variety of detergents, such as sodium laureth-4 phosphate. Personal care products incorporating cationic polysaccharides have been described in U.S. Patent 4,663,159 (Brode et al). Emulsifiers in the form of phosphate quaternary compounds have been disclosed in U.S. Patent 4,209,449, and U.S. Patent 4,503,002, each to Mayhew et al. These emulsifiers were said to be well tolerated by human tissue, exhibiting low irritation, and were stated to be suitable for use in cosmetics.

EP-A-0117035 describes detergent compositions comprising at least one anionic or amphoteric surfactant, at least one water-soluble nitrogen containing polymer and at least one water-soluble non-particulate substance. It is stated that specific cationic surfactants such as phosphotriesters may also be used in conjunction with the anionic and amphoteric surfactants although no quaternary ammonium substituted triester is exemplified. The water-soluble nitrogen containing polymer may be inter alia a cationic water-soluble quaternary nitrogen-substituted cellulose ether derivative. The only polymer of this type exemplified is polymer JR, formed by reacting hydroxyethylcellulose with a trimethyl ammonium substituted epoxide.

GB-A-2055119 relates to surfactant compositions, more particularly hair cleansing and conditioning compositions, comprising a cationic triester of phosphoric acid and at least one other surfactant which may be inter alia a cationic polymer such as hydroxyethylcellulose quaternised with trimethylamine.

Skin moisture retention has been significantly increased by utilizing many of the cationic compounds mentioned in the above patents. Nonetheless, there remains room for improvement in moisture retention. There are also the further problems of improving mildness and of providing stability against phase separation during freeze-thaw cycles.

Sunscreen formulations for use on human skin are well-known and many different types are commercially available to satisfy diverse consumer needs. For example, sunscreen formulations having different sun protection factor (SPF) values are available, thus allowing consumers to choose the amount of protection desired. SPF values range from zero upward with progressively higher values indicating increasing amounts of sun protection.

One important consideration when choosing a sunscreen is whether it resists coming off in water. Current waterproof formulations can undergo about 80 minutes in water without significant SPF loss. The formulations can be especially desirable because they eliminate the need for reapplication after swimming, bathing or excessive perspiration.

A classical method of achieving waterproof properties is through the use of a hydrophobic resin. For instance, U.S. Patent 4,897,259 (Murray et al) discloses success through use of copolymers of polyvinylpyrrolidone (PVP) and long alkyl chain olefins, commonly referred to as alkylated PVP's. Besides a sunscreen agent and the copolymer, a variety of other functional ingredients are incorporated including emulsifiers, two of which are stated to be phosphated esters and polyoxyethylene fatty ester phosphates.

Although there have been significant advances in the art of waterproofing sunscreen lotions and also improving their skin moisture retention properties, there still remains considerable room for improvement. For instance, it would be desirable to formulate a composition not including any hydrophobic resins. These resins leave undesirable residues upon the skin and also give rise to stability problems. Stability against phase separation during freeze-thaw cycles is an important goal for formulators. Finally, it is also desirable to reduce irritancy of the sunscreen products.

It is an object of at least some forms of the present invention to provide a composition in aqueous emulsion form which resists phase separation, notably under freeze-thaw cycling.

It is an object of at least some forms of the present invention to provide a cosmetic composition having improved skin moisture retention, and/or relatively low human tissue irritancy.

It is an object of some forms of this invention to provide a composition in which a sunscreen agent may be incorporated. Such a composition can avoid incorporation of a hydrophobic resin.

In a first aspect the present invention provides an aqueous composition comprising:
(i) from 0.10 to 30% of a quaternary ammonium functionalized phosphate ester wherein said phosphate ester incorporates a structure 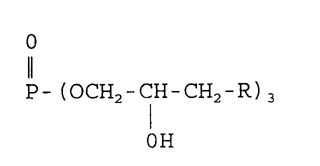 wherein R is a quaternary ammonium radical having from 5 to 40 carbon atoms; and
(ii) from 0.10 to 10% of a cationic polysaccharide substituted with a quaternary ammonium group having at least one substituent on the nitrogen being an alkyl group from 10 to 22 carbon atoms in length.

The compositions of the present invention may be either oil-in-water or water-in-oil emulsions.

Especially effective as the ester component are alkylamide quaternary ammonium phosphate esters. The cationic polysaccharide is especially effective when in the form of a cellulosic polymer quaternized with fatty alkyl groups.

In one significant form the invention also comprises a sunscreen agent. The function of such an agent is, of course, to at least partially block ultraviolet radiation from reaching human skin upon which the composition is deposited.

The present inventors have discovered that combinations of quaternary ammonium functionalized phosphate ester and cationic polysaccharide can provide an aqueous composition with some or all of several advantages. Benefits provided by various forms of this invention include freeze-thaw stability, moisture retention after water washing, and resistance to undesired removal of a sunscreen. It is not necessary for a sunscreen to include a hydrophobic resin.

A quaternary ammonium functionalized phosphate ester is a necessary first component of the compositions of this invention. The phosphate ester incorporates a quaternary ammonium radical having 5 to 40 carbon atoms of structure: 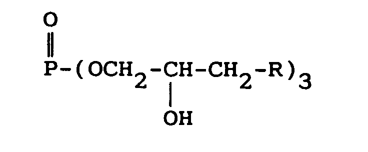 wherein R is a quaternary ammonium radical having from about 6 to about 40 carbons. This carbon atom limitation serves to include only materials of significant hydrophobic properties. The R radical can be cyclic or non-cyclic, aliphatic, aromatic or heterocyclic.

The phosphate ester may conform to a general formula 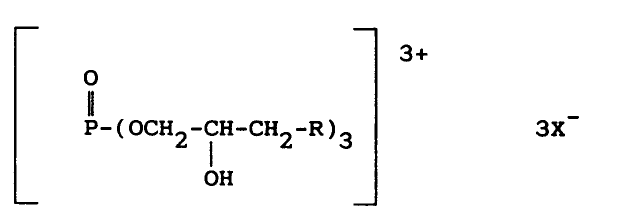 where R is as set forth above and X is an anion, such as halide, e.g., chloride.

In a preferred species, R is an amidoamine moiety of the formula: 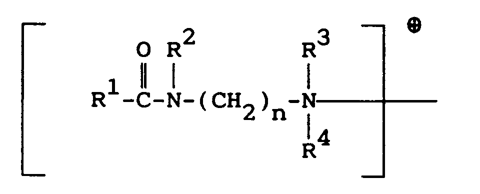 wherein
R¹ is selected from the group consisting of alkyl, alkenyl, alkoxy and hydroxyalkyl having from 5 to 22 carbon atoms each, and from aryl and alkaryl having up to 20 carbon atoms;
R², R³ and R⁴ may each independently be selected from the group consisting of hydrogen, alkyl, hydroxyalkyl and alkenyl each having up to 6 carbon atoms, and from polyoxyalkylene of up to 10 carbon atoms;
R³ and R⁴ may additionally be selected from aceto and propriono groups and may even be taken together with the nitrogen to which they are attached so as to form a N-heterocyclic ring; and
n is an integer from about 1 to 10.

In addition to the foregoing definitions where R is amidoamine, R may be a N-heterocyclic radical which may contain one additional hetero atom (e.g., oxygen or another nitrogen) and contains up to 6 total ring carbon atoms; optionally the heterocyclic radical may be substituted with alkyl and/or hydroxyalkyl of up to 20 carbon atoms each. Typical of such N-heterocyclic radicals are imidazolinyl, N-alkylmorpholino, alkylpyrimidino, alkyloxazolinyl, and the like. Such compounds may be represented by the formula: 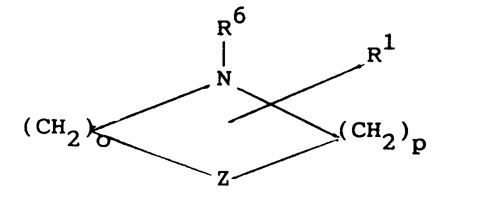 wherein
Z is N, S or O;
o is an integer from 0 to 5, especially 0 to 3;
p is an integer from 1 to 5, especially 1 to 3, provided that the sum of o + p is from 3 to 6, but especially from 3 to 4;
R₁ is a radical selected from the group consisting of alkyl, alkenyl, alkoxy and hydroxyalkyl units of from 2 to 22 carbon atoms each, and aryl and alkaryl of up to 20 carbon atoms; and
R⁶ is alkyl of from 2 to 6 carbon atoms which atoms may be substituted with a hydroxyl group.

Another possibility is that R is derived from a tertiary amine radical of from about 8 or 10 to 40 carbon atoms. More preferred are tertiary amine radicals of the type (C₆-C₂₀ alkyl, dimethyl) amine such as N,N-dimethyl myristylamine, N,N-dimethyl-palmityl-amine, and N,N-dimethyl-laurylamine.

The phosphate esters identified as Formulas I through III below are of particular interest. These are commercially available from Mona Industries, Inc., Paterson, New Jersey, sold under the Monaquat designation.

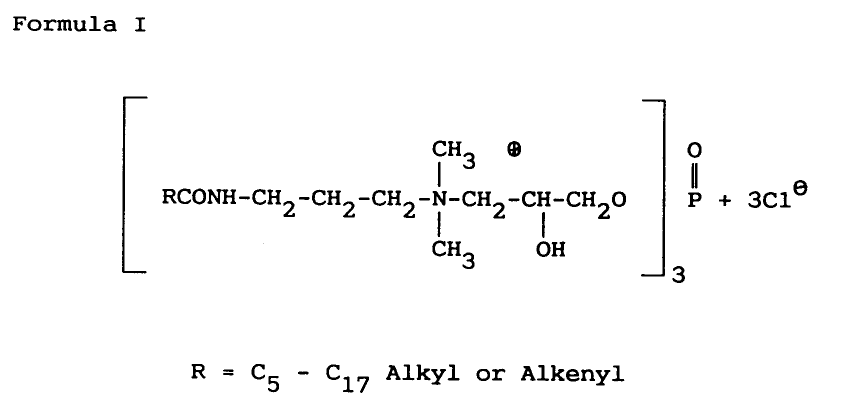

Compounds within this formula include those commercially available as Monaquat P-TC, P-TD, P-TS and phospholipid EFA. 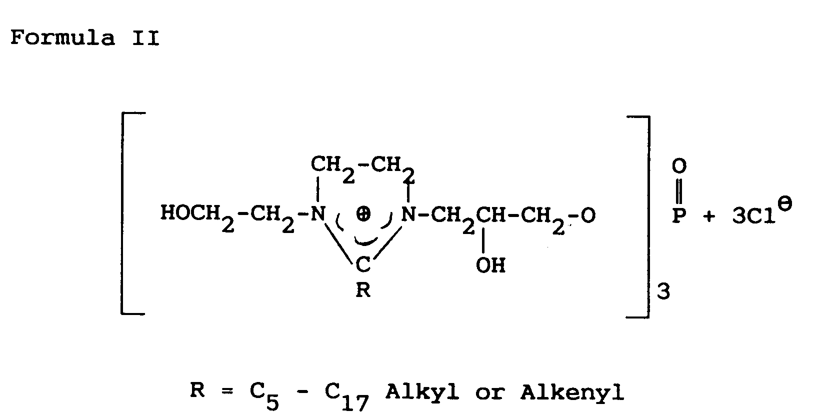 This compound is commercially available as Monaquat P-TZ.

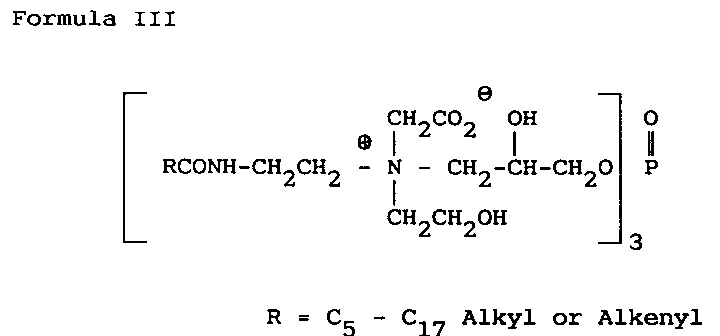

This compound is commercially available as Monaquat P-TL.

The chain lengths of the groups R, and the content of ester of the stated formula (% active), in the commercially available esters is given in the following table:-

| Monaquat | Alkyl Group | % Active |
|---|---|---|
| P-TC | C₅ - C₁₇ | 40.0 |
| P-TD | C₁₁ - C₁₃ | 34.0 |
| P-TL | C₁₁ - C₁₃ | 30.0 |
| P-TS | C₁₇ | 30.0 |
| P-TZ | C₅ - C₁₇ | 30.0 |
| Phospholipid EFA | C₁₇ with 2 double bonds | 30.0 |

Most preferred from the above compounds are Monaquat P-TS, Monaquat P-TC, Monaquat P-TD and Phospholipid EFA.

Amounts of quaternary phosphate ester will range from about 0.1 to about 30%, preferably from about 1 to about 15%, optimally between about 2 and 10% by weight of the composition.

A second essential component of the composition of this invention is a cationic polysaccharide which incorporates hydrophobic substituent groups e.g. hydrocarbyl groups having 10 or more carbon atoms. Polysaccharides of this invention are in general naturally occurring polysaccharides or those modified by etherification, which are quaternized with a nitrogen-containing compound and alkylated with a compound, which may be a nitrogen-containing compound, containing a hydrophobe. A hydrophobe is at least one substituent on nitrogen which is an alkyl group having from 10, preferably 12 up to 22 preferably 20 carbon atoms.

Polysaccharide starting materials include the naturally occurring, biosynthesized and derivatized carbohydrate polymers or mixtures thereof. Such materials encompass high molecular weight polymers composed of monosaccharide units joined by glycosidic bonds. These materials include the entire starch and cellulose families, pectin, chitosan; chitin; the seaweed products such as agar and carrageenan; alginate; the natural gums such as guar, arabic and tragacanth; bio-derived gums such as xanthan; and the like. Preferred starting materials include cellulosics conventionally employed for the preparation of cellulose ethers, such as chemical cotton, cotton linters, wood pulp, alkali cellulose, and the like and ether derivatives of the same. Such cellulose ethers include hydroxyethyl cellulose, hydroxypropyl cellulose, methyl cellulose, carboxymethyl cellulose, carboxyethyl cellulose, hydroxypropyl methyl cellulose, hydroxyethyl methyl cellulose, hydroxyethyl carboxymethyl cellulose, and the like. A particularly preferred polysaccharide starting material is hydroxyethyl cellulose. The polysaccharide starting material will possess a molecular weight corresponding to the number of polysaccharide repeat units, usually from 50 up to about 20,000. The molecular weight of the polysaccharides may be varied through controlled degradation procedures known in the art.

Etherified polysaccharides may be obtained commercially or produced from the polysaccharide starting materials mentioned previously. Etherification involves reacting pendent hydroxyl groups on the polysaccharide backbone with an etherifying agent, or mixtures thereof, which contain functional groups reactive with such hydroxyl groups. Etherification may be conducted to enhance the water-solubility of the polysaccharides, e.g. by ethoxylation. Typical etherifying agents include lower alkylating agents such as dimethyl sulfate, diethyl sulfate, methyl chloride, methyl bromide, ethyl chloride, ethyl bromide or n-propyl chloride; hydroxy alkylating agents such as ethylene oxide, propylene oxide or glycidol; and carboxy alkylating agents such as monochloroacetic acid, sodium chloroacetate or chloropropionic acid.

The polysaccharide starting materials are, in general, provided with quarternary nitrogen-containing substituents through quaternization reactions. Quaternization may be achieved by reacting the polysaccharides with quaternizing agents which are quaternary ammonium salts, including mixtures thereof, to effect substitution of the polysaccharide chain with quaternary nitrogen-containing groups. Typical quaternary ammonium salts which can be utilized include quaternary nitrogen-containing halides, halohydrins and epoxides. The quaternary ammonium salt may contain hydrophobes.

Particularly preferred are polymeric quaternary ammonium salts of hydroxyethyl cellulose reacted with a fatty alkyl dimethyl ammonium substituted epoxide. Illustrative preferred materials in this category are available from Union Carbide Corporation under the trademark Quatrisoft LM-200 and from Croda Inc under the trademark Crodacel Q (QL, QM and QS).

These materials have substituents on the polysaccharide chain which are of the general formula 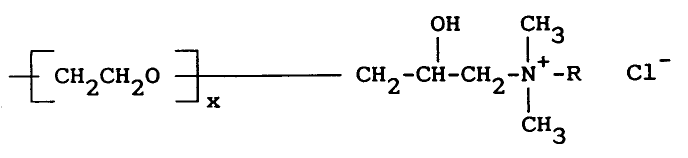 in which R is fatty alkyl, preferably having a chain length of 10 to 20 carbon atoms. 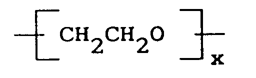 denotes a residue from the hydroxyethyl substitution on the cellulose.

The alkyl groups R are as follows:
- Crodacel QL: Lauryl
- Crodacel QM: Coco
- Crodacel QS: Stearyl
- Quatrisoft LM-200: Lauryl

Amounts of the cationic polysaccharide will normally range from about 0.01 to about 10%, preferably from abut 0.01 to about 5%, optimally between about 0.2 and 1% by weight of the compositions.

Although the invention is not limited to cosmetic compositions in the form of emulsions, a particularly suitable vehicle is that of an emulsion. By definition, an emulsion is a dispersed system containing at least two immiscible liquid phases, one of which is dispersed in the form of small droplets throughout the other. Water and oil are the most common immiscible liquid phases. An emulsion in which oil is dispersed as droplets throughout the aqueous phase is termed an oil-in-water emulsion. When water is the dispersed phase and an oil is the dispersion medium, a water-in-oil emulsion exists. Contemplated within the scope of this invention are emulsions in the forms of lotions and creams of both types of emulsions, those where the water phase is continuous and those where the oil phase is continuous. The ratios of these phases may range from about 99:1 to 1:99 by weight.

Although the quaternary ammonium functionalized phosphate esters are intended as the primary emulsifier and surfactant for systems of this invention, there may also be present nonionic emulsifiers. Examples of satisfactory nonionic emulsifiers include fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono and di-fatty acid esters of ethylene glycol wherein the fatty acid moiety contains from 10 to 20 carbon atoms, fatty acid monoglyceride wherein the fatty acid moiety contains from 10 to 20 carbon atoms, diethylene glycol, polyethylene glycols of molecular weight 200 to 6000, propylene glycol of molecular weight 200 to 3000, sorbitol, sorbitan, polyoxyethylene sorbitol, polyoxyethylene sorbitan, and hydrophilic wax esters. Amounts of the nonionic emulsifier may range anywhere from about 0.1 to about 20% by weight of the emulsion, preferably from about 2 to about 10% by weight.

If a composition of this invention is intended to serve as a sunscreen, then a sunscreen agent is a necessary component of the compositions of this invention. The term "sunscreen agent" as used herein defines ultraviolet ray-blocking compounds exhibiting absorption within the wavelength region between 290 and 420 nm. Sunscreens may be classified into five groups based upon their chemical structure: para-amino benzoates; salicylates; cinnamates; benzophenones; and miscellaneous chemicals including menthyl anthralinate and digalloyl trioleate. Inorganic sunscreens may also be used including titanium dioxide, zinc oxide, iron oxide and polymer particles such as those of polyethylene and polyamides. Preferred materials include p-aminobenzoic acid and its derivatives, anthralinates; salicylates; cinnamates; courmarin derivatives; azoles; and tannic acid and its derivatives. Among FDA approved sunscreens are those listed in the table below.

| UV-A Absorbers | Approved % |
|---|---|
| Oxybenzone, also known as 2-hydroxy-4-methoxy benzophenone, and benzophenone-3, available as Uvinul M-40 and Gafsorb 2H4M | |
| | 2-6 |
| Dioxybenzone, also known as 2,2 dihydroxy-4-methoxy benzophenone, and benzophenone-8 | 3 |
| Sulibenzone, also known as 2-hydroxy-4-methoxy benzophenone-5-sulphonic acid, and benzophenone-4, available as Uvinul MS-40 and Gafsorb 2H4MS | |
| | 5-10 |
| Menthyl anthralinate, also known as menthyl-o-aminobenzoate | |
| | 3.5-5 |

| UV-B Absorbers | Approved % |
|---|---|
| p-Amino benzoic acid, also known as PABA | 5-15 |
| Amyl dimethyl PABA (NA), also known as amyl-p-dimethyl ammonium benzoate, available as Padimate A | 1-5 |
| 2-Ethoxy ethyl p-methoxy cinnamate (NA), available as Cinoxate and Givtan-F | 1-3 |
| Diethanolamine p-ethoxy cinnamate, also known as DEA methoxy cinnamate, available as Parsol-Hydro | 8-10 |
| Digalloyl trioleate (NA), a component of Solprotex I | 2-5 |
| Ethyl-4-bis (hydroxypropyl) aminobenzoate, also known as ethyl dihydroxy propyl PABA, available as Amerscreen P | 1-5 |
| 2-Ethyl hexyl-2-cyano-3,3 diphenyl acrylate, also known as octocrylene and available as Uvinul N-539 | 7-10 |
| Ethyl hexyl p-methoxy cinnamate, also known as octyl methoxycinnamate available as Parsol MCX | 2-7.5 |
| 2-Ethyl hexyl salicylate, also known as octyl salicylate | 3-5 |
| Glyceryl aminobenzoate, also known as glyceryl p-aminobenzoate and glyceryl PABA, available as Escalol 106 | 2-3 |
| Homomenthyl salicylate, also known as 3,3,5-trimethylcyclohexyl salicylate | 4-15 |
| Lawsone with dihydroxyacetone (NA) | 0.25 with 33 |
| Octyl dimethyl PABA, also known as 2-ethyl hexyl p-dimethyl p-aminobenzoate, and 2-ethyl hexyl dimethyl PABA, available as Padimate O and Escalol 507 | 1.4-8 |
| 2-Phenyl benzimidazole 5-sulphoic acid | 1.4 |
| Triethanolamine salicylate | 5-12 |

| Physical Screens | Approved % |
|---|---|
| Red Petrolatum | 30-100 |
| Titanium dioxide | 2-25 |

Emollient cosmetic compositions embodying this invention, but also other product forms such as sunscreens, may include a variety of oily emollients. These emollients may be selected from one or more of the following classes:
1. Hydrocarbon oils and waxes. Examples thereof are mineral oil, petrolatum, paraffin, ceresin, ozokerite, microcrystalline wax, polyethylene, and perhydrosqualene.
2. Triglyceride esters such as vegetable and animal fats and oils. Examples include caster oil, cocoa butter, safflower oil, cottonseed oil, corn oil, olive oil, cod liver oil, almond oil, avocado oil, palm oil, sesame oil, squalane, and soybean oil.
3. Acetoglyceride esters, such as acetylated monoglycerides.
4. Ethoxylated glycerides, such as ethoxylated glyceryl monostearate.
5. Alkyl esters of fatty acids having 10 to 20 carbon atoms. Methyl, isopropyl, and butyl esters of fatty acids are useful herein. Examples include hexyl laurate, isohexyl laurate, isohexyl palmitate, isopropyl palmitate, decyl oleate, isodecyl oleate, hexadecyl stearate, decyl stearate, isopropyl isostearate, diisopropyl adipate, diisohexyl adipate, dihexyldecyl adipate, diisopropyl sebacate, lauryl lactate, myristyl lactate, and cetyl lactate.
6. Alkenyl esters of fatty acids having 10 to 20 carbon atoms. Examples thereof include oleyl myristate, oleyl stearate, and oleyl oleate.
7. Fatty acids having 10 to 20 carbon atoms. Suitable examples include pelargonic, lauric, myristic, palmitic, stearic, isostearic, hydroxystearic, oleic, linoleic, ricinoleic, arachidic, behenic, and erucic acids.
8. Fatty alcohols having 10 to 20 carbon atoms. Lauryl, myristyl, cetyl, hexadecyl, stearyl, isostearyl, hydroxystearyl, oleyl, ricinoleyl, behenyl, erucyl, and 2-octyl dodecanyl alcohols are examples of satisfactory fatty alcohols.
9. Fatty alcohol ethers. Ethoxylated fatty alcohols of 10 to 20 carbon atoms including the lauryl, cetyl, stearyl, isostearyl, oleyl, and cholesterol alcohols, having attached thereto from 1 to 50 ethylene oxide groups or 1 to 50 propylene oxide groups.
10. Ether-esters such as fatty acid esters of ethoxylated fatty alcohols.
11. Lanolin and derivatives. Lanolin, lanolin oil, lanolin wax, lanolin alcohols, lanolin fatty acids, isopropyl lanolate, ethoxylated lanolin, ethoxylated lanolin alcohols, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, ethoxylated sorbitol lanolin, and liquid and semisolid lanolin absorption bases are illustrative of emollients derived from lanolin.
12. Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 mono- oleate, polypropylene glycol 2000 monostearate, ethoxylated propylene glycol monostearate, glyceryl mono- and di-fatty acid esters, polyglycerol poly-fatty esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters.
13. Wax esters such as beeswax, spermaceti, myristyl myristate, stearyl stearate.
14. Beeswax derivatives, e.g. polyoxyethylene sorbitol beeswax. These are reaction products of beeswax with ethoxylated sorbitol of varying ethylene oxide content, forming a mixture of ether esters.
15. Vegetable waxes including carnauba and candelilla waxes.
16. Phospholipids such as lecithin and derivatives.
17. Sterols. Cholesterol, cholesterol fatty acid esters are examples thereof.
18. Amides such as fatty acid amides, ethoxylated fatty acid amides, solid fatty acid alkanolamides.

Amounts of the above listed emollients may range anywhere from about 0.5 to about 40% by weight of the total composition. Preferably the amounts of these emollients will range from about 2 to about 25%, optimally between about 5 and 15% by weight.

Humectants of the polyhydric alcohol-type may also be included in the compositions of this invention. The humectant aids in increasing the effectiveness of the emollient, reduces scaling, stimulates removal of built-up scale and improves skin feel. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, 1,2,6-hexanetriol, ethoxylated glycerol, propoxylated glycerol and mixtures thereof. For best results the humectant is preferably glycerol. The amount of humectant may range anywhere from 0.5 to 20%, preferably between 1 and 15% by weight of the composition.

For improved lubricity, there may also be included one or more silicone oils or fluids which may be selected from a dimethyl polysiloxane, a methylphenyl polysiloxane and an alcohol-soluble silicone glycol copolymer., Preferred siloxanes include dimethyl polysiloxane (CTFA name dimethicone), a polysiloxane end-blocked with trimethyl units and polydimethylcyclosiloxane, (CTFA name cyclomethicone). The preferred siloxanes exhibit a viscosity from about 2 to 50 centistokes at 25°C. Amounts of the silicones can range up to 30% by weight of the compositions, preferably from about 1 to about 10% by weight.

The emulsions of the invention can also include thickeners/viscosifiers in amounts up to about 5% by weight of the composition. As known to,those skilled in the art, the precise amount of thickeners can vary depending upon the consistency and thickness of the composition which is desired. Exemplary thickeners are xanthan gum, sodium carboxymethyl cellulose, hydroxyalkyl and alkyl celluloses, and cross-linked acrylic acid polymers such as those sold by B.F. Goodrich under the Carbopol trademark.

Preservatives can desirably be incorporated into the cosmetic compositions of this invention to protect against the growth of potentially harmful microorganisms. While it is in the aqueous phase that microorganisms tend to grow, microorganisms can also reside in the oil phase. As such, preservatives which have solubility in both water and oil are preferably employed in the present compositions. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are methyl paraben, imidazolidinyl urea, sodium dehydroxyacetate, propyl paraben and benzyl alcohol. The preservatives should be selected having regard for the use of the composition and possible incompatibilities between the preservatives and other ingredients in the emulsion. Preservatives are preferably employed in amounts ranging from about 0.01% to about 2% by weight of the composition.

Amounts of water in the composition may range anywhere from about 1 to about 99%, preferably from about 40 to about 90%, optimally between about 60 and 85% by weight.

Minor adjunct ingredients may also include fragrances, antifoam agents, bacteriostats, opacifiers and colorants, each in their effective amounts to accomplish their respective functions.

The following examples will more fully illustrate the embodiments of this invention. All parts, percentages and proportions referred to herein arid in the appended claims are by weight unless otherwise indicated.

### EXAMPLE 1

An emulsion typical of the present invention is provided in Table I.

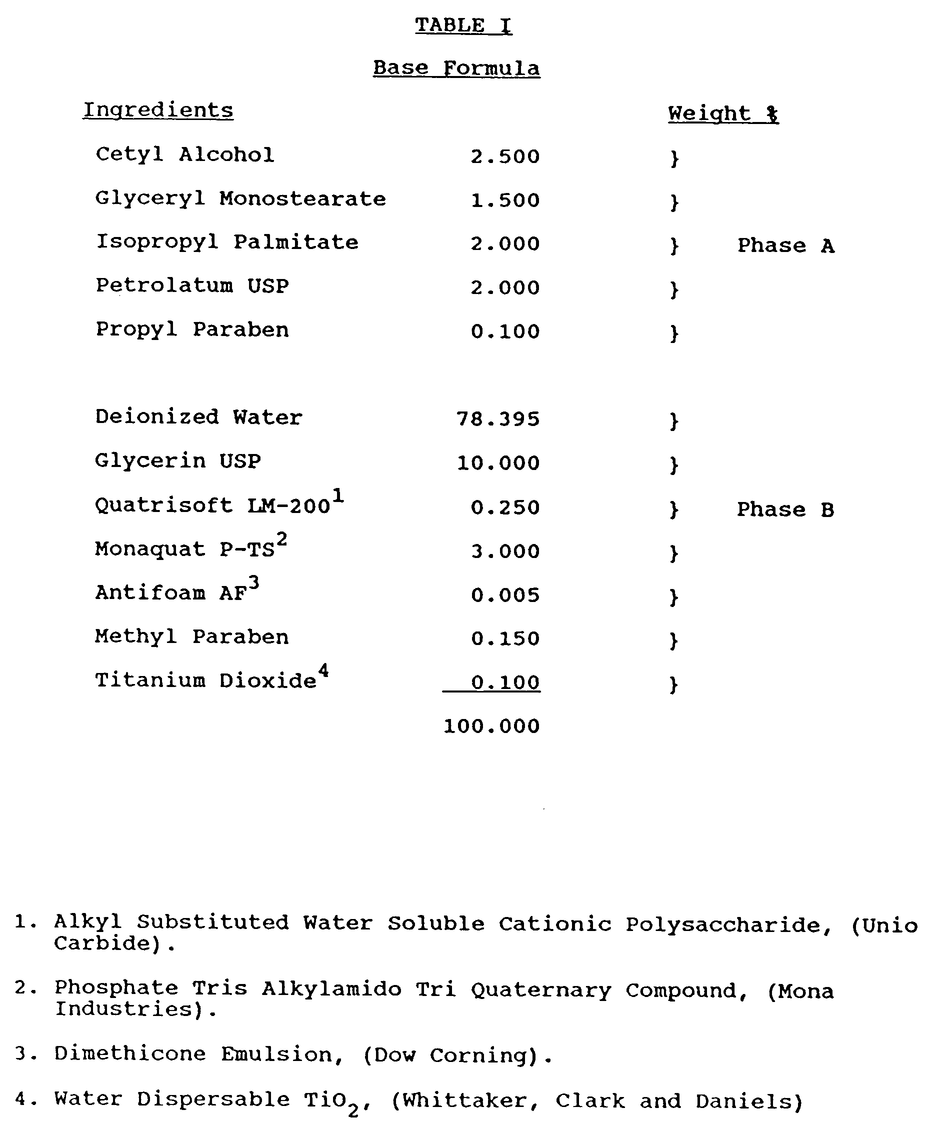

The base formula outlined in Table I was prepared by first dispersing Quatrisoft LM-200 in glycerin. Thereafter, the Monaquat P-TS and water were combined into the glycerin dispersion. The resultant mixture was heated under moderate stirring at 70°C. Methyl paraben, titanium dioxide and antifoam AF were then added to the aqueous Phase B.

Separately prepared was an oil Phase A wherein cetyl alcohol, glyceryl monostearate, isopropyl palmitate, petrolatum USP and propyl paraben were combined under heating at 70°C. Phase A was then added to Phase B with moderate stirring. The combined phases were then homogenized until a white emulsion was achieved. This emulsion was then cooled to 32°C with stirring.

### EXAMPLE 2

The Base Formula of Example 1 was modified by omitting the cationic polymer, and by replacing it with various other cationic polymers. The resulting emulsions were investigated for their freeze-thaw properties. These formulations were subjected to three freeze-thaw cycles from -18°C to 22°C and their appearances were recorded under Table II.

**Table II**

| No. | Polymer* | Freeze-Thaw Cycles | | |
|---|---|---|---|---|
| | | 1 | 2 | 3 |
| 1 | None | Grainy | Grainy | Grainy |
| 2 | Quatrisoft LM200 | Smooth | Smooth | Smooth |
| 3 | Polymer JR-400 | Grainy | Grainy | Grainy |
| 4 | Polymer JR-125 | Grainy | Grainy | Grainy |
| 5 | Polymer JR-30M | Grainy | Grainy | Grainy |
| 6 | Jaquar C-13S | Smooth | Smooth | Grainy |
| 7 | HyCare 1000 | Smooth | Grainy | Grainy |
| 8 | Celquat H-100 | Slightly Grainy | Slightly Grainy | Grainy |
| 9 | Celquat L-200 | Grainy | Grainy | Grainy |
| 10 | Crodacel Q | Smooth | Smooth | Smooth |

| | | | | |
|---|---|---|---|---|
| * All polymers were incorporated at 0.25% weight active level. | | | | |

From the results listed under Table II, it is seen that certain types of cationic polymers exhibit much better freeze-thaw stability. Particularly effective are Quatrisoft LM200 and Crodacel Q. On the other hand, cationic polymers such as the Polymer JR series exhibit graininess. Polymers in the JR series, also known as polyquaternium 10 and as Quaternium 19, are hydroxyethylcellulose reacted with trimethyl ammonium substituted propylene oxide.

### EXAMPLE 3

The Base Formula of Example 1 was modified by substituting a series of different quaternary ammonium functionalized phosphate esters. The resulting emulsions were evaluated with respect to freeze-thaw cycle properties. Each of the emulsion formulations incorporated 3% of the phosphate ester. Table III lists the results of the freeze-thaw cycle tests.

**Table III**

| No. | Phosphate Ester | Freeze-Thaw Cycles | | |
|---|---|---|---|---|
| | | 1 | 2 | 3 |
| 1 | Monaquat P-TS | Smooth | Smooth | Smooth |
| 2 | Monaquat P-TC | Smooth | Smooth | Smooth |
| 3 | Monaquat P-TD | Smooth | Smooth | Smooth |
| 4 | Monaquat P-TL | Grainy | Grainy | Grainy |
| 5 | Monaquat P-TZ | Grainy | Grainy | Grainy |
| 6 | Phospholipid EFA | Smooth | Smooth | Smooth |

Evident from Table III is that the Base Formula with either Monaquat P-TS, P-TC, or P-TD of Phospholipid EFA provides excellent freeze-thaw cycle properties. Relatively poor performance in terms of graininess was exhibited by Monaquat P-TL and P-TZ.

### EXAMPLE 4

The Porcine Skin Moisturization Assay was used to evaluate a series of commercial hand and body lotions, formulations of the present invention and comparative formulations omitting either the cationic polysaccharide or the phosphate ester. This test was used to detect differences in the moisture absorption/desorption and resistance to wash-off of lotions on porcine skin.

The test involved accurate weighing of 6 uniform pieces of porcine skin. These pieces were hydrated and then the test lotion applied. Periodically, the skin pieces were weighed at their reduced relative humidity. Directly after each weighing the pieces were uniformly rinsed in water and weighed once again under reduced relative humidity. Graphs of weight versus time were plotted for both pre-rinse and post-rinse results. Total areas under the dry-out curve before and after rinsing were then determined. Table IV below lists results of these evaluations.

Test lotion 5 was the base formula quoted in Example 1. Test lotion 4 was the same except for a reduced glycerol content. Test lotions 6, 7 and 8 differed from the test lotion in that a constituent was omitted or replaced, as stated in Table IV.

**Table IV**

| No. | TEST LOTION | TOTAL AREA PRE-RINSE | TOTAL AREA POST-RINSE |
|---|---|---|---|
| 1 | Eversoft® | 1990.8 | -615.8 |
| 2 | Lubriderm® | 2268.5 | 1154.4 |
| 3 | Keri Lotion® | 1659.8 | -1008.5 |

| Test | Cationic Lotion 5% Glycerin | | |
|---|---|---|---|
| 4 | Monaquat P-TS/Quatrisoft LM-200 | 3535.8 | 1947.2 |

| Test | Cationic Lotion 10% Glycerin | | |
|---|---|---|---|
| 5 | Monaquat P-TS/Quatrisoft LM-200 | 4560.0 | 1965.7 |
| 6 | Monaquat P-TS/Crodacel QL | 3865.4 | 3337.4 |
| 7 | Monaquat P-TS/No Polymer | 3865.4 | 3512.2 |

| Test | Nonionic Lotion 10% Glycerin | | |
|---|---|---|---|
| 8 | No Monaquat/Quatrisoft LM-200 | 3391.3 | 587.18 |

Test lotions utilizing Monaquat P-TS exhibited post-rinse area results which were superior to competitive products. Poor results were obtained when only Quatrisoft LM-200 was utilized in the absence of any Monaquat.

### EXAMPLE 5

Sunscreen compositions embodying the present invention are set out in Tables V and VI below. In Table V organic sunscreen agents are incorporated as the sunscreen agent. In Table VI an inorganic sunscreen agent, namely micronized titanium dioxide, is incorporated.

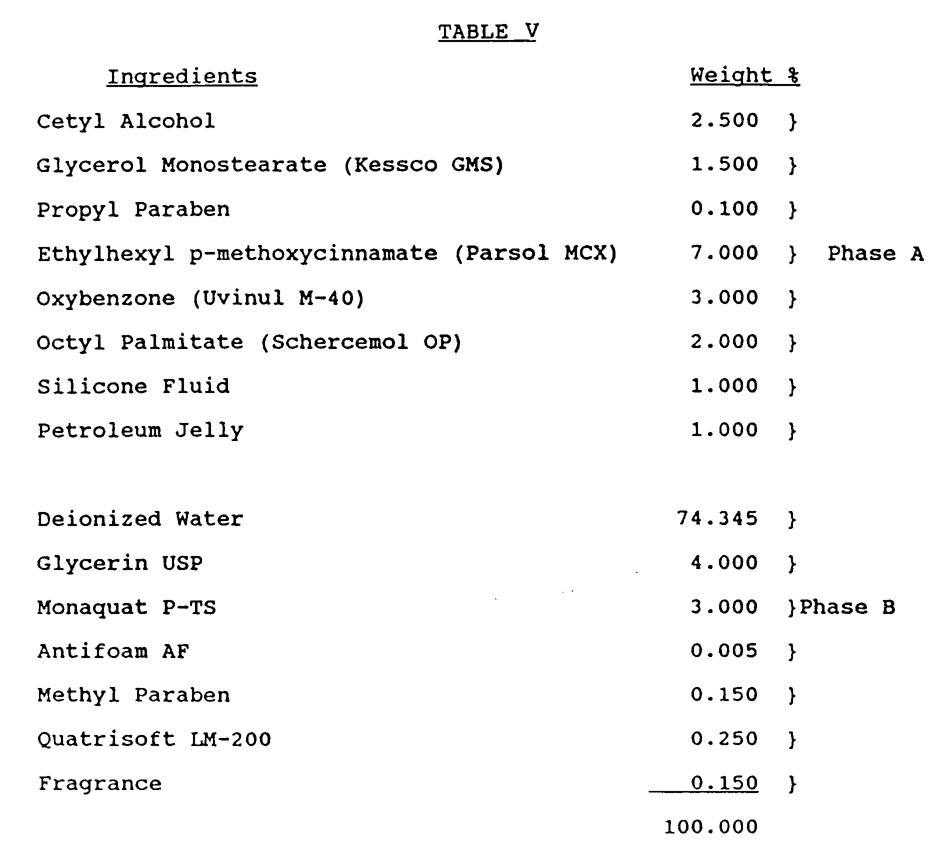

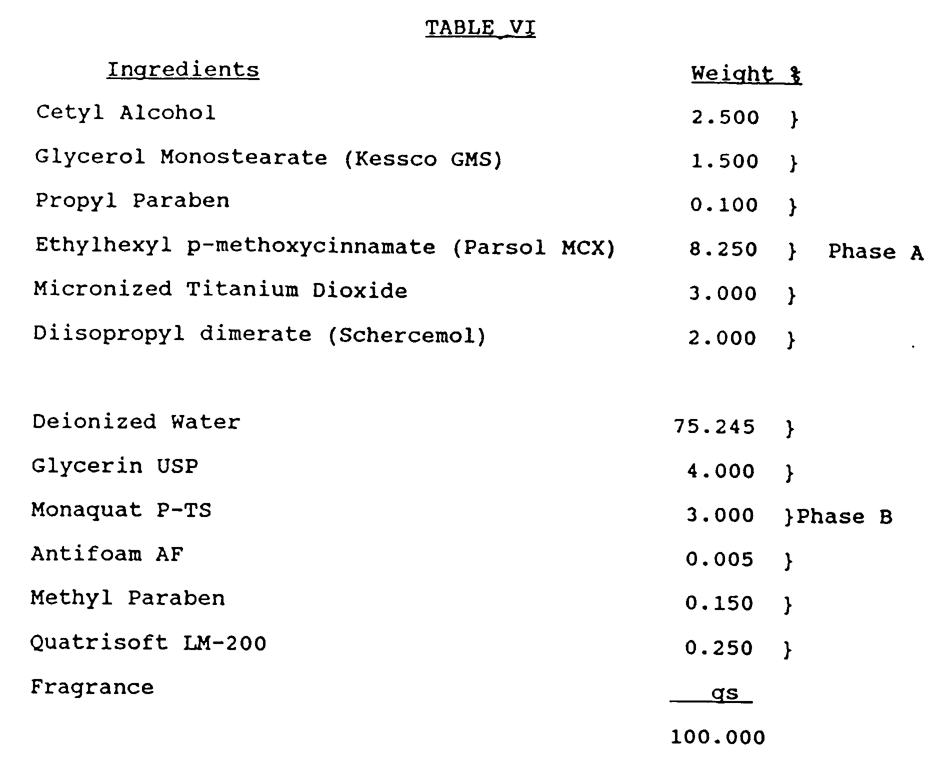

### EXAMPLE 6

The lotions set out in Tables V and VI were subjected to five member Sun Protection Factor (SPF) tests on human skin. These tests complied with a monograph of the United States Food and Drug Administration (FDA). The results obtained were:

| Lotion | UV absorbers | SPF before swim | SPF after swim |
|---|---|---|---|
| Table V | Ethylhexyl p-methoxycinnamate Oxybenzone | 16.65 | 15.15 |
| Table VI | Ethylhexyl p-methoxycinnamate Micronized Titanium Dioxide | 17.14 | 16.44 |

It can be seen from these results that the lotions embodying the invention retain a high proportion of their efficacy even after exposure to water during swimming.

## Claims

1. An aqueous composition comprising:
(i) from 0.1 to 30% of a quaternary ammonium functionalised phosphate ester wherein said phosphate ester incorporates a structure 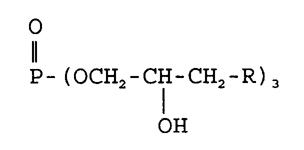 wherein R is a quaternary ammonium radical having from 5 to 40 carbon atoms; and
(ii) from 0.1 to 10% of a cationic polysaccharide substituted with a quaternary ammonium group having at least one substituent on the nitrogen being an alkyl group from 10 to 22 carbon atoms in length.

2. A composition according to claim 1 wherein R is an amidoamine having the formula: 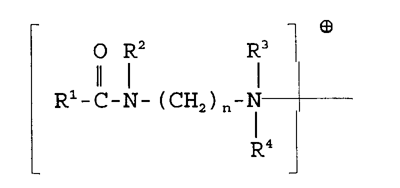 wherein
R¹ is selected from the group consisting of alkyl, alkenyl, alkoxy and hydroxyalkyl having from 5 to 22 carbon atoms each, and from aryl and alkaryl having up to 20 carbon atoms;
R², R³ and R⁴ may each independently be selected from the group consisting of hydrogen, alkyl, hydroxyalkyl and alkenyl each having up to 6 carbon atoms, and from polyoxylene of up to 10 carbon atoms;
R³ and R⁴ may additionally be selected from aceto and propiono groups and may even be taken together with the nitrogen to which they are attached so as to form a N-heterocyclic ring; and
n is an integer from 1 to 10.

3. A composition according to claim 1 wherein when R is a N-heterocyclic ring containing one additional hetero atom, represented by the formula: 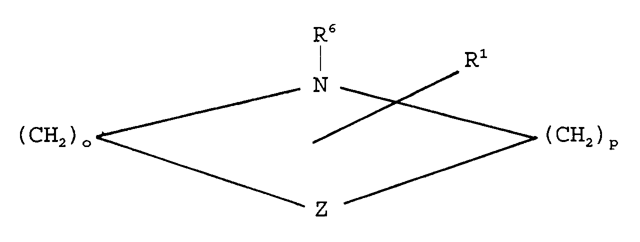 wherein
Z is N, S or O;
o is an integer from 0 to 5,
p is an integer from 1 to 5, provided that the sum of o+p is from 3 to 6;
R₁ is a radical selected from the group consisting of alkyl, alkenyl, alkoxy and hydroxyalkyl units of from 2 to 22 carbon atoms each, and aryl and alkaryl of up to 20 carbon atoms; and
R⁶ is alkyl of from 2 to 6 carbon atoms which alkyl may be substituted with a hydroxyl group.

4. A composition according to any one of claims 1 to 3 wherein the alkyl substituent on the nitrogen of quaternary ammonium group of cationic polysaccharide is selected from the group consisting of lauryl, coco and stearyl radicals.

5. A composition according to any one of the preceding claims wherein said phosphate ester is present in an amount from 1 to 5% by weight of the composition.

6. A composition according to any one of the preceding claims wherein said cationic polysaccharide is present in an amount from 0.2 to 1% by weight of the composition.

7. A composition according to any one of the preceding claims wherein the phosphate ester is present as the main emulsifier and surfactant of the composition.

8. A composition according to any one of the preceding claims further comprising a substance having UV absorption in the wavelength region between 290 and 420 nanometres, as a sunscreen agent.

9. A composition according to claim 8 wherein the sunscreen agent is selected from the group consisting of para-amino benzoates; salicylates; cinnamates; benzophenones; methyl anthralinates, digalloyl trioleates, courmarin derivatives; azoles; tannic acid and its derivatives, inorganic pigments, water soluble polymeric particles and mixtures thereof.

10. A composition according to claim 8 wherein the sunscreen agent is selected from the group consisting of oxybenzone, dioxybenzone, sulibenzone, menthyl anthralinate, p-amino benzoic acid, amyl dimethyl p-aminobenzoate acid, 2-ethoxy ethyl p-methoxy cinnamate, diethanolamine p-ethoxy cinnamate, digalloyl trioleate ethyl-4-bis (hydroxypropyl) aminobenzoate, 2-ethyl hexyl-2-cyano-3, 3 diphenyl acrylate, ethyl hexyl p-methoxy cinnamate, 2-ethyl hexyl salicylate, glyceryl aminobenzoate, homomenthyl salicylate, dihydroxyacetone, octyl dimethyl p-aminobenzoic acid, 2-phenyl benzimidazole 5-sulphonic acid, triethanolamine salicylate, titanium dioxide, iron oxide, polyethylene, polyamide and mixtures thereof.

## Patentansprüche

1. Wäßrige Zusammensetzung, die die folgenden Bestandteile umfaßt:
(i) 0,1 bis 30% eines durch quaternäre Ammoniumgruppen funktionalisierten Phosphatesters, wobei der Phosphatester die folgende Struktur besitzt: 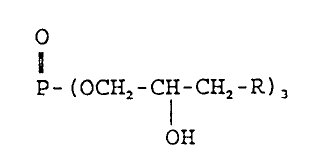 worin R für einen quaternären Ammoniumrest mit 5 bis 40 Kohlenstoffatomen steht, und
(ii) 0,1 bis 10% eines mit einer quaternären Ammoniumgruppe mit mindestens einem Substituenten am Stickstoff in Form einer Alkylgruppe mit einer Länge von 10 bis 22 Kohlenstoffatomen substituierten kationischen Polysaccharids.

2. Zusammensetzung nach Anspruch 1, wobei R ein Amidoamin der folgenden Formel ist: 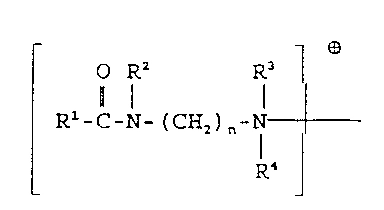 worin R¹ unter Alkyl, Alkenyl, Alkoxy und Hydroxyalkyl mit jeweils 5 bis 22 Kohlenstoffatomen und aus Aryl und Alkaryl mit bis zu 20 Kohlenstoffatomen ausgewählt ist, R², R³ und R⁴ jeweils unabhängig voneinander unter Wasserstoff, Alkyl, Hydroxyalkyl und Alkenyl mit jeweils bis zu 6 Kohlenstoffatomen und aus Polyoxylen mit bis zu 10 Kohlenstoffatomen ausgewählt sein können, R³ und R⁴ des weiteren aus Aceto- und Propionogruppen ausgewählt sein können und selbst zusammen mit dem Stickstoffatom, an dem sie hängen, einen N-Heterozyklus bilden können, und
n eine ganze Zahl von 1 bis 10 bedeutet.

3. Zusammensetzung nach Anspruch 1, wobei R ein N-Heterozyklus mit einem weiteren Heteroatom der folgenden Formel ist: 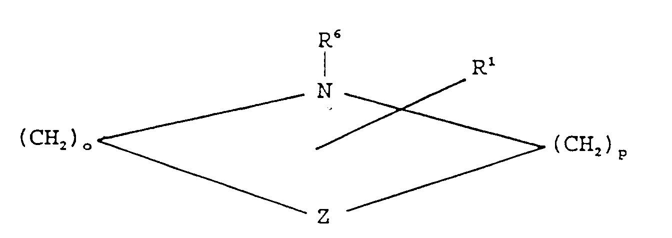 worin bedeuten:
Z N, S oder O;
o eine ganze Zahl von 0 bis 5,
p eine ganze Zahl von 1 bis 5, wobei gilt, daß die Summe aus o und p 3 bis 6 ist;
R¹ ein unter Alkyl-, Alkenyl-, Alkoxy- und Hydroxyalkyleinheiten mit jeweils 2 bis 22 Kohlenstoffatomen und Aryl- und Alkaryl mit bis zu 20 Kohlenstoffatomen ausgewählter Rest und
R⁶ Alkyl mit 2 bis 6 Kohlenstoffatomen, wobei das Alkyl durch eine Hydroxylgruppe substituiert sein kann.

4. Zusammensetzung nach einem der Ansprüche 1 bis 3, wobei der Alkylsubstituent am Stickstoff der quaternären Ammoniumgruppe des kationischen Polysaccharids unter Lauryl-, Kokosnuß- und Stearylresten ausgewählt ist.

5. Zusammensetzung nach einem der vorhergehenden Ansprüche, wobei der Phosphatester in einer Menge von 1 bis 5 Gew.-% der Zusammensetzung vorhanden ist.

6. Zusammensetzung nach einem der vorhergehenden Ansprüche, wobei das kationische Polysaccharid in einer Menge von 0,2 bis 1 Gew.-% der Zusammensetzung vorhanden ist.

7. Zusammensetzung nach einem der vorhergehenden Ansprüche, wobei der Phosphatester als Hauptemulgator und grenzflächenaktives Mittel der Zusammensetzung vorhanden ist.

8. Zusammensetzung nach einem der vorhergehenden Ansprüche, die des weiteren eine Substanz mit UV-Absorption in einem Wellenlängenbereich von 290 bis 420 nm als Sonnenschutzmittel umfaßt.

9. Zusammensetzung nach Anspruch 8, wobei das Sonnenschutzmittel unter para-Aminobenzoaten, Salicylaten, Cinnamaten, Benzophenonen, Methylanthralinaten, Digalloyltrioleaten, Coumarinderivaten, Azolen, Gerbsäure und Derivaten hiervon, anorganischen Pigmenten, wasserlöslichen Polymerteilchen und Gemischen hiervon ausgewählt ist.

10. Zusammensetzung nach Anspruch 8, wobei das Sonnenschutzmittel unter Oxybenzon, Dioxybenzon, Sulibenzon, Menthylanthralinat, p-Aminobenzoesäure, Amyldimethyl-p-aminobenzoatsäure, 2-Ethoxyethyl-p-methoxycinnamat, Diethanolamin-p-ethoxycinnamat, Digalloyltrioleat, Ethyl-4-bis-(hydroxypropyl)-aminobenzoat, 2-Ethylhexyl-2-cyano-3,3-diphenylacrylat, Ethylhexyl-p-methoxycinnamat, 2-Ethylhexylsalicylat, Glycerylaminobenzoat, Homomenthylsalicylat, Dihydroxyaceton, Octyldimethyl-p-Aminobezoesäure, 2-Phenylbenzimidazol-5-sulfonsäure, Triethanolaminsalicylat, Titandioxid, Eisenoxid, Polyethylen, Polyamid und Gemischen hiervon ausgewählt ist.

## Revendications

1. Composition aqueuse comprenant:
(i) de 0,10 à 30% d'un ester de phosphate d'ammonium quaternaire fonctionnalisé, dans lequel ledit ester de phosphate comporte une structure 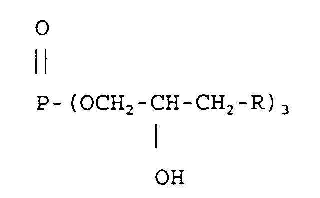 dans laquelle R est un radical ammonium quaternaire ayant de 5 à 40 atomes de carbone; et
(ii) de 0,10 à 10% d'un polysaccharide cationique remplacé par un groupe ammonium quaternaire dont au moins un remplaçant de l'azote est un groupe alkyle d'une longueur de 10 à 22 atomes de carbone.

2. Composition selon la revendication 1, dans laquelle R est un amidoamine ayant pour formule: 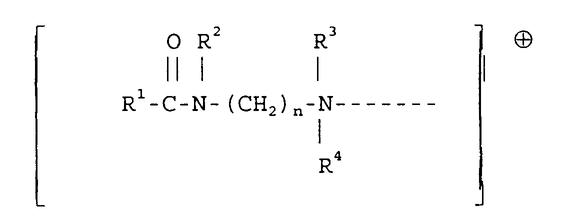 dans laquelle
R¹ est choisi dans le groupe constitué de alkyle, alkényle, alkoxy et hydroxyalkyle, ayant de 5 à 22 atomes de carbone chacun, et de aryle et alkaryle, ayant jusqu'à 20 atomes de carbone.
R², R³ et R⁴ peuvent être choisis chacun indépendamment dans le groupe constitué de hydrogène, alkyle, hydroxyalkyle et alkényle, ayant chacun jusqu'à 6 atomes de carbone, et de polyoxyalkylène ayant jusqu'à 10 atomes de carbone;
R³ et R⁴ peuvent de plus, être choisis dans les groupes acéto et propriono, et peuvent même être pris avec l'azote auquel ils sont fixés de façon à former un anneau N-hétérocylique; et
n est un entier de 1 à 10.

3. Composition selon la revendication 1, dans laquelle R est un radical N-hétérocyclique contenant un hétéro atome supplémentaire, représenté par la formule: 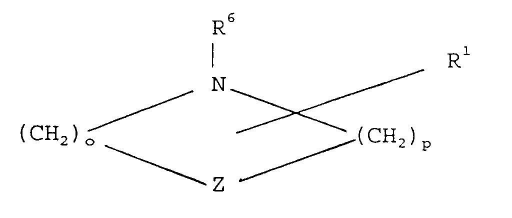 dans laquelle
Z est N, S ou O;
o est un entier allant de 0 à 5;
p est un entier allant de 1 à 5, en particulier de 1 à 3, à condition que la somme de o + p aille de 3 à 6;
R₁ est un radical choisi dans le groupe constitué d'unités alkyle, alkényle, alkoxy et hydroxyalkyle, ayant de 2 à 22 atomes de carbone chacune, et aryle et alkaryle allant jusqu'à 20 atomes de carbone; et
R⁶ est un alkyle ayant de 2 à 6 atomes de carbone, lequel alkyle pouvant être remplacé par un groupe hydroxyle.

4. Composition selon l'une quelconque des revendications 1 à 3, dans laquelle le substituant alkyle sur l'azote du groupe ammonium quaternaire de polysaccharide cationique est choisi dans le groupe constitué de radicaux lauryl, coco et stéaryl.

5. Composition selon l'une quelconque des revendications précédentes, dans laquelle ledit ester de phosphate est présent dans une quantité allant de 1 à 5% en poids de la composition.

6. Composition selon l'une quelconque des revendications précédentes, dans laquelle ledit polysaccharide cationique est présent dans une quantité allant de 0,2 à 1% en poids de la composition.

7. Composition selon l'une quelconque des revendications précédentes, dans laquelle l'ester de phosphate est présent en tant qu'émulsifiant et tensioactif principal de la composition.

8. Composition selon l'une quelconque des revendications précédentes, comprenant en outre une substance ayant une absorption d'UV dans la région des longueurs d'ondes comprises entre 290 et 420 nanomètres, en tant qu'antisolaire.

9. Composition selon la revendication 8, dans laquelle l'antisolaire est choisi dans le groupe constitué de para-amino benzoates; salicylates; cinnamates; benzophénones; méthyl anthralinates, digalloyl trioléates, dérivés de courmarin; azoles; acide tannique et ses dérivés, pigments inorganiques, particules polymères solubles dans l'eau et leurs mélanges.

10. Composition selon la revendication 8, dans laquelle l'antisolaire est choisi dans le groupe constitué de oxybenzone, dioxybenzone, sulibenzone, menthyl anthralinate, acide p-amino benzoique, acide amyl diméthyl p-aminobenzoate, 2-éthoxy éthyle p-méthoxy cinnamate, diéthanolamine p-éthoxy cinnamate, digalloyl trioléate éthyl-4-bis (hydroxypropyle) aminobenzoate, 2-éthyl hexyl-2-cyano-3, 3 diphényle acrylate, éthyl hexyl p-méthoxy cinnamate, 2-éthyl hexyl salicylate; glycéryl aminobenzoate, homomenthyl salicylate, dihydroxyacétone, octyl diméthyl acide p-aminobenzoique, 2-phényl benzimidazole acide 5-sulphonique, triéthanolamine salicylate, dioxyde de titane, oxyde de fer, polyéthylène, polyamide et leurs mélanges.
